# EUROPEAN PATENT SPECIFICATION

(11) **EP 0 455 899 B1**
(45) Date of publication and mention of the grant of the patent: **16.02.2000**
(21) Application number: 90305046.6
(22) Date of filing: 10.05.1990
(51) Int. Cl.: C07C 405/00, A61K 31/557

(54) **Prostaglandin I analogue**
Prostaglandin-I-Analoga
Analogues de prostaglandine I

(43) Date of publication of application: 13.11.1991
(73) Proprietor: R-TECH UENO, LTD., Osaka-shi, Osaka-fu (JP)
(72) Inventor: Ueno, Ryuzo, Hyogo-ken (JP); Ueno, Ryuji, Hyogo-ken (JP); Oda, Tomio, Hyogo-ken (JP)
(74) Representative: Atkinson, Peter Birch

(56) References cited:
- EP-A- 0 134 153
- GB-A- 2 013 661
- GB-A- 2 017 699

## Description

### BACKGROUND OF THE INVENTION

The present invention relates to a novel compound, a prostaglandin I analogue, which is referred to as PGIs simply hereinafter.

It has been well known that PGI₂ has a platelet aggregation controlling effect and a hypotensive activity, but it simultaneously has some side effects such as increasing of pulse rate, hot flush, abdominalgia and the like (S.M.M. Karim et al, PC. Med., 9,307(1982)).

Japanese Patent Application KOKAI No. 61-78734 discloses an effectivity of tricarbonyl-aromatic-group VIB metals complex as a catalyst for reduction of α,β-unsaturated carbonyl compound, and the production of {(1S,2R,3R,5S)-(E)-7-(4-carbomethoxybutylidene)-2-(3-oxooctyl)-3-tetrahydropyranyloxy-bicyclo[3.3.0]octane} by the reaction of {(1S,5S,6S,7R)-3-(4-carbomethoxy-1(EZ)-butenyl)-6-(3-oxo-(E)-1-octenyl)-7-tetrahydropyranyloxy-bicyclo[3.3.0]octane} in the Example 7. Further, Japanese Patent Application KOKAI No. 61-37740 discloses the same compounds as the above in the Example 7. However, any prior arts as referred do not disclose the PGIs (I) and (II) of the present invention, and any usefulness thereof.

GB-A-2 013 661 discloses 9-deoxy-9a methylene isosteres of PGI₂ which are stated to have pharmacological actions similar to those of natural prostacyclin. As such, the compounds may be used for treatment of asthma or stomach ulcers and for induction of labour in females. Included in the disclosure of GE-A-2 013 661 are various 16-fluoro compounds but these are not specifically disclosed as having either a hypotensive activity or an inhibitory effect on platelet aggregation.

### SUMMARY OF THE INVENTION

The present invention provides a novel compound of PGIs represented by the following formula (I) and (II): 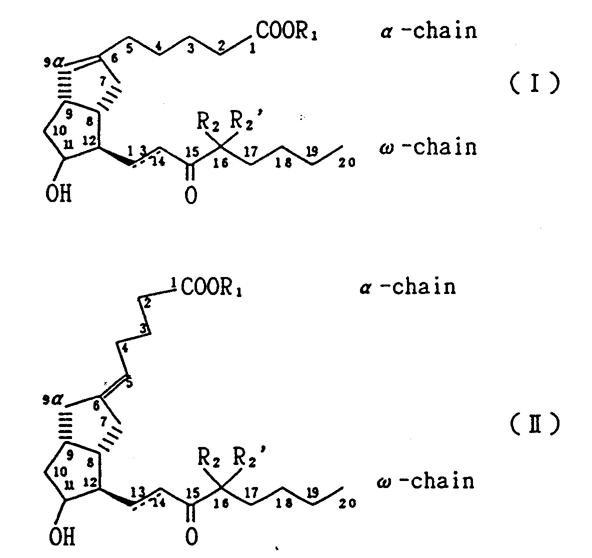 wherein R₁ is a hydrogen atom or an alkyl group, R₂ is a fluorine atom, and R₂' is a hydrogen atom, a fluorine atom, an alkyl group, a phenyl group, a benzyl group, a hydroxyl group or an alkoxy group or salts thereof.

The PGIs represented by the formula (I) are generally named as 15-keto-16-substituted-9(O)-methano-Δ^{6(9α)}-PGI₁s or 13,14-dihydro-15-keto-16-substituted-9(O)-methano-Δ^{6(9α)}-PGI₁s; and the PGIs represented by the formula (II) are generally named as 15-keto-16-substituted-9(O)-methano-PGI₂ or 13,14-dihydro-15-keto-16-substituted-9(O)-methano-PGI₂s.

The PGIs of the present invention is characterized by a bicyclic ring in the molecular skeleton and by one or two specific substituents at the 16-position. Such substituent(s) contributes to a blood pressure hypotension, and reduction of side effects such as increase of pulse rate, and other pharmaceutical or physiological activities of PGIs.

A group represented by R₂' includes a hydrogen atom, a fluorine atom, an alkyl group such as methyl, and ethyl; a phenyl group; a benzyl group; a hydroxyl group; or an alkoxy group such as methoxy, and ethoxy. Preferably R₂' is a fluorine atom.

R₁ in the present invention is a hydrogen atom or alkyl group. As the alkyl group methyl, ethyl, n-propyl, isopropyl, n-butyl, t-butyl group and the like are included. When R₁ is a hydrogen atom, corresponding carboxyl group may form a suitable salt. When the compound of the present invention is used for a pharmaceutical composition, the salt may be a physiologically acceptable one. Alkali forming such a salt typically includes an alkaline metal such as sodium, potassium and the like, an alkaline earth metal such as calcium, magnesium and the like, ammonia, alkylamine, alkanolamine such as monoethanolamine, diethanolamine, triethanolamine, propanolamine and the like, alkylalkanolamine and the like.

The 13,14-dihydro-PGI₁s represented by (I) and saturated at the C₁₃-C₁₄ bond can be prepared, for instance, according to the Synthetic Scheme [I]. That is, commercially available (1S,5S,6R,7R)-6-(trialkylsiloxymethyl)-3-formyl-7-tetrahydropyranyloxy-bicyclo[3.3.0]octo-2-ene (aldehyde compound (1)) is reacted with ylide which is separately prepared from (3-carboxypropyl)triphenylphosphine bromide and potassium t-butoxide, and then the resultant is reacted with diazomethane to give an ester (2). The ester (2) is subjected with tetra n-butylammonium fluoride to remove the silyl group to yield the alcohol (3). This alcohol (3) is subjected to Collins oxidation to give the aldehyde (4), which is then reacted with an anion prepared from dimethyl(2-oxo-3-substituted-heptyl)phosphonates and sodium hydride so as to introduce ω-chain. The double bond in the ω-chain is hydrogenated with palladium/carbon and the like under hydrogen atmosphere. In this process a double bond in ω-chain, if there is, is hydrogenated, but a double bond in the ring is not. The tetrahydropyranyl group, a protective group, is removed with an acid to give an ester (7) of an objective compound (I). An acid corresponding to the ester (7) can be obtained by hydrolysis of the ester (7) according to a usual work-up. Though as an example of phosphonates which can be used for introduce of ω-chain one having a fluorine atom at the 3-position as a substituent is illustrated in the Example 1, this substituent may be a halogen such as a chlorine atom; or others such as a methyl, ethyl, phenyl, benzyl, hydroxyl, methoxy or ethoxy group and the like.

PGI₁s represented by the formula (I) and having a double bond between C₁₃-C₁₄ can be prepared according to a process illustrated by the Synthetic Scheme [II]. In this process the ester (2) which can be prepared according to the same manner as in the Scheme [I] is hydrogenated using palladium/carbon under hydrogen atmosphere to give 4-carbomethoxybutyl compounds (2'). This compound (2') is subjected with tetra n-butylammonium fluoride to remove the silyl group to yield the alcohol (3'). This alcohol (3') is subjected to Collins oxidation to give the aldehyde (4'), which is then reacted with an anion which is prepared from dimethyl(2-oxo-3-substituted-heptyl)phosphonates and sodium hydride to introduce ω-chain to give the 15-keto compound (5'). The tetrahydropyranyl group, a protective group, is removed with an acid to give an ester (7') of an objective compound (I). An acid corresponding to the ester (7') can be obtained by hydrolysis of the ester (7') according to a usual work-up. Though as an example of phosphonates which can be used for introduction of ω-chain one having a fluorine atom at the 3-position as a substituent is illustrated in the Example 4, this substituent may be a halogen atom such as a chlorine atom; or other groups such as a methyl, ethyl, phenyl, benzyl, hydroxyl, methoxy or ethoxy group and the like.

16,16-Difluoro compound (9) can be prepared by reacting an anion derived from dimethyl(2-oxo-3,3-difluoroheptyl)phosphonate with the aldehyde (4') as illustrated in the Synthetic Scheme [III].

13,14-Dihydro-PGI₂s represented by the formula (II) can be prepared according to the Synthetic Scheme [IV]. The compound (5) which can be prepared according to the same manner as illustrated in the Scheme [I] can be hydrogenated using tricarbonyl chromium methyl benzoate complex (refer to Japanese Patent Application KOKAI No.61-37740) (in this case the two double bonds on the α-chain and in the ring, which conjugate each other are also hydrogenated to one double bond between the carbon atoms bonding the ring and the α-chain). The obtained compound is treated with an acid to remove the tetrahydropyranyl group to yield an ester (15') of the objective compound (II). Alternatively, the compound (11) obtained in the above process is reduced with sodium borohydride to alcohol, and then the alcohol is hydrolyzed with an alkali to give a carboxylic acid (13). The objective carboxylic acid (15) can be obtained by removing the tetrahydropyranyl group by hydrolysis after Jones oxidation. In the Scheme [IV] a fluorine atom is shown as a substituent on the carbon atom adjacent to the carbonyl group, but another substituent as explained hereinbefore may be used.

PGIs represented by the formula (II) can be prepared from the compound (5) which can be obtained according to the processes illustrated the Synthetic Scheme [V]. The carbonyl group of the compound (5) is reduced using sodium borohydride to give a 15-hydroxy compound (5''), which compound (5'') is then hydrogenated using tricarbonyl chromium benzoic acid methyl complex (see Japanese Patent Application KOKAI No. 61-37740) (in this case two double bonds on the α-chain and in the ring, which conjugates each other are also hydrogenated to one double bond between the carbon atoms bonding the ring and the α-chain).

The obtained compound (12) was hydrolyzed with alkali to an acid (13'), which acid (13') is then oxidized by Jones oxidation to give a ketone (14'). From the ketone (14') is removed the tetrahydropyranyl group by an acid to yield the objective carboxylic acid (15'). The substituent(s) on the carbon atom adjucent to the carbonyl group may be other atom(s) or group(s) as aforementioned.

16,16-Difluoro compound (21) can be prepared by reacting an anion derived from dimethyl(2-oxo-3,3-difluoroheptyl)phosphonate with aldehyde (4) as illustrated in the Synthetic Scheme [VI].

The PGIs of the present invention may include isomers or mixture of isomers. As isomers there are exemplified a keto-hemiacetal tautomers between hydroxyl group at 11-position and carbonyl group at 15-position and optical isomers, geometric isomers and the like.

The present invention is illustrated by Examples, in which compounds are nominated according to IUPAC excepting final objective compounds. The carbon number of the bicyclic ring is indicated as follows:

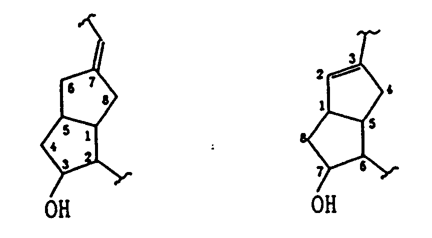

### Example 1

Preparation of 13,14-dihydro-15-keto-16(RS)-fluoro-9(O)-methano-Δ^{6(9α)}-PGI₁ methyl ester:

### 1-1 Synthesis of (1S,5S,6S,7R)-6-(t-butyldimethylsiloxymethyl)-3-[4-carbomethoxy-1(EZ)-butenyl]-7-tetrahydropyranyloxy-bicyclo[3.3.0]octo-2-ene (2):

Commercially available (1S,5S,6S,7R)-6-(t-butyldimethylsiloxymethyl)-3-formyl-7-tetrahydropyranyloxy-bicyclo[3.3.0]octo-2-ene (1.00 g) (1) was reacted with ylide which was prepared from (3-carboxypropyl)triphenylphosphine bromide and potassium t-butoxide. A crude carboxylic acid was obtained according to a usual work-up. The product was reacted with diazomethane in ether. A crude product obtained after a usual work-up was purified on a column chromatography (hexane/ethyl acetate = 10/1) to give (1S,5S,6S,7R)-6-(t-butyldimethylsiloxymethyl)-3-[4-carbomethoxy-1(EZ)-butenyl]-7-tetrahydropyranyloxy-bicyclo[3.3.0]octo-2-ene (2) as a colorless oily product.
Yield: 0.85 g (67%)
¹H NMR (CDCl₃) δ 0.05 (6H,s), 0.90 (9H,s), 1.05 - 1.95 (10H,m), 2.10 - 3.13 (7H,m), 3.27 - 4.22 (5H,m), 3.63 (3H,s), 4.45 - 4.69 (1H,m), 5.05 - 5.65(2H,m), 5.97 (0.67H,d, J = 12 Hz), 6.22 (0.33H, d, J = 16 Hz)

### 1-2 Synthesis of (1S,5S,6S,7R)-3-[4-carbomethoxy-1(EZ)-butenyl]-6-hydroxymethyl-7-tetrahydropyranyloxy-bicyclo[3.3.0]octo-2-ene (3):

(1S,5S,6S,7R)-6-(t-Butyldimethylsiloxymethyl)-3-[4-carbomethoxy-1(EZ)-butenyl]-7-tetrahydropyranyloxy-bicyclo[3.3.0]octo-2-ene (2) obtained in 1-1 (0.85 g) was dissolved in THF, into which tetra-n-butylammonium fluoride in THF (1.1 M, 6.43 ml) was added, and the mixture was stirred for 18 hours. A crude product obtained after a usual work-up was purified on column chromatography (hexane/ethyl acetate = 1/1) to give (1S,5S,6S,7R)-3-[4-carbomethoxy-1(EZ)-butenyl]-6-hydroxymethyl-7-tetrahydropyranyloxy-bicyclo[3.3.0]octo-2-ene (3) as a colorless oily product. Yield : 0.59 g (96%)
¹H NMR (CDCl₃)δ 1.18 ∼ 1.93(10H,m), 2.16 ∼ 3.28(8H,m), 3.42 ∼ 4.07(5H,m), 3.63(3H, s), 4.55 ∼ 4.64(0.5H,m), 4.66 ∼ 4.77(0.5H,m), 5.33(0.67H, dt, J = 7.5 Hz, J = 12.5 Hz), 5.42 ∼ 5.67(1.33H,m), 5.99(0.67H,d,J = 12.5 Hz), 6.26(0.33H,d,J = 15.5 Hz).

### 1-3 Synthesis of (1S,5S,6S,7R)-3-[4-carbomethoxy-1(EZ)-butenyl]-6-[4(RS)-fluoro-3-oxo-(E)-1-octenyl]-7-tetrahydropyranyloxy-bicyclo[3.3.0]octo-2-ene (5):

(1S,5S,6S,7R)-3-[4-Carbomethoxy-1(EZ)-butenyl]-6-hydroxymethyl-7-tetrahydropyranyloxy-bicyclo[3.3.0]octo-2-ene (3) (0.240 g) was subjected to Collins oxidation in methylene chloride at 0 °C. Into the reaction mixture was added sodium hydrogen sulfonate, and filtered. A crude aldehyde (4) obtained after concentration under reduced pressure of the filtrate was dissolved in THF, and reacted with an anion which was prepared from dimethyl (2-oxo-3-fluoroheptyl) phosphonate (0.61 g) and sodium hydride as stirring at 50 °C for 5 hours. The reaction mixture was neutralized with acetic acid. A crude product obtained after a usual work-up was purified on column chromatography (hexane/ethyl acetate = 6/1) to give (1S,5S,6S,7R)-3-[4-carbomethoxy-1(EZ)-butenyl]-6-[4(RS)-fluoro-3-oxo-(E)-1-octenyl]-7-tetrahydropyranyloxy-bicyclo[3.3.0]octo-2-ene (5) as a pale yellow oily product. Yield: 0.250 g (85%)
¹H NMR(CDCl₃)δ 0.70 ∼ 1.07(3H,m), 1.06 ∼ 2.14(15H,m), 2.15 ∼ 4.16(11H,m), 3.66(3H,s), 4.43 ∼ 4.72(1.5H,m), 4.96 ∼ 5.71(2.5H,m), 5.95(0.67H,d,J = 11 Hz), 6.24(0.33H,d,J = 16 Hz), 6.36 ∼ 6.73(1H,m), 6.83 ∼ 7.23(1H,m).

### 1-4 Synthesis of(1S,5S,6R,7R)-3-(4-carbomethoxybutyl)-6-[4(RS)-fluoro-3-oxo-octyl]-7-tetrahydropyranyloxy-bicyclo[3.3.0]octo-2-ene (6):

(1S,5S,6S,7R)-3-[4-Carbomethoxy-1(EZ)-butenyl]-6-[4(RS)-fluoro-3-oxo-(E)-1-octenyl]-7-tetrahydropyranyloxy-bicyclo[3.3.0]octo-2-ene (5) (0.094 g) was dissolved in ethyl acetate, into which palladium(5 wt %)/carbon (0.0094 g) was added and stirred under hydrogen atmosphere at 15 °C for 2 hours. The reaction mixture was filtered, and the filtrate was evaporated under reduced pressure. Obtained crude product was chromatographed using a silica gel treated with silver nitrate (15 wt %) (hexane/ethyl acetate = 12/1 - 9/1) to give (1S,5S,6R,7R)-3-(4-carbomethoxybutyl)-6-[4(RS)-fluoro-3-oxo-octyl]-7-tetrahydropyranyloxybicyclo[3.3.0]octo -2-ene (6) as a pale yellow oily product. Yield: 0.042 g (44%)
¹H NMR(CDCl₃)δ 0.66 ∼ 1.03(3H,m), 1.03 ∼ 3.12(31H,m), 3.24 ∼ 4.02(3H,m), 3.63(3H,s), 4.27 ∼ 4.53(0.5H,m), 4.50 ∼ 4.70(1H,m), 4.83 ∼ 5.06(0.5H,m), 5.06 ∼ 5.33(1H,m).

### 1-5 Synthesis of 13,14-dihydro-15-keto-16(RS)-fluoro-9(O)-methano-Δ^{6(9α)}-PGI₁ methyl ester:

(1S,5S,6R,7R)3-(4-Carbomethoxybutyl)-6-[4(RS)-fluoro-3-oxo-octyl]-7-tetrahydropyranyloxy-bicyclo[3.3.0]octo-2-ene (6) (0.088 g) was dissolved in a mixture of acetic acid, water and THF (4 : 2 : 1) and stirred at 45 °C for 4 hours. A reaction mixture was concentrated under reduced pressure, and a crude product obtained was purified on column chromatography (hexane/ethyl acetate = 6/1 - 4/1) to give 13,14-dihydro-15-keto-16(RS)-fluoro-9(O)-methano-Δ^{6(9α)}-PGI₁ methyl ester (7) as a pale yellow product. Yield: 0.072 g (100%)
¹H NMR(CDCl₃)δ 0.73 ∼ 1.05(3H,m), 1.05 ∼ 3.15(26H,m), 3.46 ∼ 4.04(1H,m), 3.63(3H,s), 4.33 ∼ 4.56(0.5H,m), 4.48 ∼ 5.07(0.5H,m), 5.07 ∼ 5.36(1H,m).

### Example 2

### Preparation of 13,14-dihydro-15-keto-16(RS)-fluoro-9(O)-methano-PGI₂:

### 2-1 Synthesis of (1S,2R,3R,5S)-7(E)-(4-carbomethoxybutylidene)-2-[4(RS)-fluoro-3-oxo-octyl]-3-tetrahydropyranyloxy-bicyclo[3.3.0]octane (11):

(1S,5S,6S,7R)-3-[4-Carbomethoxy-1(EZ)-butenyl]-6-[4(RS)-fluoro-3-oxo-(E)-1-octenyl]-7-tetrahydropyranyloxy-bicyclo[3.3.0]octo-2-ene (5) (0.109 g) was dissolved in acetone in an autoclave, into which a tricarbonyl chromium/methyl benzoate complex (0.023 g) was added. The mixture was degassed and the content was stirred under a hydrogen atmosphere (70 kg/cm²) at 125 °C for 20 hours. The reaction mixture was concentrated under reduced pressure. A crude product obtained was chromatographed (hexane/ethyl acetate = 10/1 - 7/1 to give (1S,2R,3R,5S)-7(E)-(4-carbomethoxybutylidene)-2-[4(RS)-fluoro-3-oxo-octyl]-3-tetrahydropyranyloxy-bicyclo[3.3.0]octane (11) as a colorless oily product. Yield: 0.157 g (99%)
¹H NMR(CDCl₃)δ 0.76 ∼ 1.05(3H,m), 1.05 ∼ 2.91(31H,m), 3.27 ∼ 3.98(3H,m), 3.62(3H,s), 4.31 ∼ 4.72(1.5H,m), 4.79 ∼ 5.32(1.5H,m).

### 2-2 Synthesis of (1S,2R,3R,5S)-7(E)-(4-carbomethoxybutylidene)-2-[4(RS)-fluoro-3(RS)-hydroxyoctyl]-3-tetrahydropyranyloxybicyclo[3.3.0]octane (12):

(1S,2R,3R,5S)-7(E)-(4-Carbomethoxybutylidene)-2-[4(RS)-fluoro-3-oxo-octyl]-3-tetrahydropyranyloxy- bicyclo[3.3.0]octane (11) (0.197 g) was dissolved in methanol, to which a sodium borohydride (0.017 g) was added at 0 °C. The mixture was stirred for 30 minutes and treated as a usual work-up. A crude product obtained was chromatographed (hexane/ethyl acetate = 3/1) to give (1S,2R,3R,5S)-7(E)-(4-carbomethoxybutylidene)-2-[4(RS)-fluoro-3(RS)-hydroxy-octyl]-3-tetrahydropyranyloxybicyclo[3.3.0]octane (12).
Yield: 0.185 g (93%)
¹H NMR(CDCl₃)δ 0.72 ∼ 1.05(3H,m), 1.05 ∼ 2.66(32H,m), 3.22 ∼ 4.15(4.5H,m), 3.62(3H,s), 4.42 ∼ 4.67(1.5H,m), 5.00 ∼ 5.31(1H,m).

### 2-3 Synthesis of (1S,2R,3R,5S)-7(E)-(4-Carboxybutylidene)-2-[4(RS)-fluoro-3(RS)-hydroxyoctyl]-3-tetrahydropyranyloxybicyclo[3.3.0]octane (13):

(1S,2R,3R,5S)-7(E)-(4-Carbomethoxybutylidene)-2-[4(RS)-fluoro-3(RS)-hydroxy-octyl]-3-tetrahydropyranyloxybicyclo[3.3.0]octane (12) (0.185 g) was dissolved in methanol, to which an aqueous solution of 1N sodium hydroxide (6.5 ml) was added. The mixture was stirred at room temperature for 4 hours. After a usual work-up a crude product (1S,2R,3R,5S)-7(E)-(4-carboxybutylidene)-2-[4(RS)-fluoro-3(RS)-hydroxyoctyl]-3-tetrahydropyranyloxybicyclo[3.3.0]octane (13) was obtained. Yield: 0.184 g

### 2-4 Synthesis of (1S,2R,3R,5S)-7(E)-(4-carboxybutylidene)-2-[4(RS)-fluoro-3-oxooctyl]-3-tetrahydropyranyloxybicyclo[3.3.0]octane (14):

(1S,2R,3R,5S)-7(E)-(4-Carboxybutylidene)-2-[4(RS)-fluoro-3(RS)-hydroxyoctyl]-3-tetrahydropyranyloxybicyclo[3.3.0]octane (13) (0.184 g) was oxidized with Jones reagent between about -15 and -5 °C. After stirring for 40 minutes, isopropyl alcohol (0.43 ml) was added, and treated by a usual work-up. Obtained crude product was purified on column chromatography (hexane/ethyl acetate = 15/1 - 10/1) using silica gel treated with an acid (CC-4: available from Mallineckrodt Co., Ltd.) to give (1S,2R,3R,5S)-7(E)-(4-carboxybutylidene)-2-[4(RS)-fluoro-3-oxooctyl]-3-tetrahydropyranyloxybicyclo[3.3.0]octane (14) as a colorless oily product. Yield: 0.072 g (40%)
¹H NMR(CDCl₃)δ 0.72 ∼ 1.04(3H,m), 1.04 ∼ 2.88(31H,m), 3.20 ∼ 3.98(3H,m), 4.20 ∼ 4.68(1.5H,m), 4.75 ∼ 5.33(1.5H,m), 6.52 ∼ 8.52(1H,brs).

### 2-5 Preparation of 13,14-dihydro-15-keto-16(RS)-fluoro-9(O)-methano-PGI₂ (15):

(1S,2R,3R,5S)-7(E)-(4-Carboxybutylidene)-2-[4(RS)-fluoro-3-oxooctyl]-3-tetrahydropyranyloxybicyclo[3.3.0]-octane (14) (0.070 g) was dissolved in a mixture of acetic acid, water and THF (4 : 2 : 1) and stirred at 45 °C for 3.5 hours. The reaction mixture was concentrated under reduced pressure. A crude product obtained was purified on column chromatography (hexane/ethyl acetate = 3.5/1) using a silica gel (CC-4) to give 13,14-dihydro-15-keto-16(RS)-fluoro-9(O)-methano-PGI₂ (15) as a colorless oily product. Yield: 0.048 g (84%)
¹H NMR(CDCl₃)δ 0.65 ∼ 1.05(3H,m), 1.05 ∼ 2.85(25H,m), 3.43 ∼ 3.82(1H,m), 4.26 ∼ 4.57(0.5H,m), 4.76 ∼ 5.35(1.5H,m), 5.20 ∼ 6.57(2H,brs).

### Example 3

### Preparation of 13,14-dihydro-15-keto-16(RS)-fluoro-9(O)-methano-PGI₂ methyl ester (15'):

(1S,2R,3R,5S)-7(E)-(4-Carbomethoxybutylidene)-2-[4(RS)-fluoro-3-oxo-octyl]-3-tetrahydropyranyloxybicyclo[3.3.0]octane (11) (0.070 g) was dissolved in a mixture of acetic acid, water and THF (4 : 2 : 1), and stirred at 45 °C for 3 hours. The reaction mixture was concentrated under reduced pressure, and crude product obtained was purified on column chromatography (hexane/ethyl acetate = 3.5/1) to give 13,14-dihydro-15-keto-16(RS)-fluoro-9(O)-methano-PGI₂ methyl ester (15') as a colorless oily product. Yield: 0.038 g (67 %)
¹H NMR(CDCl₃) δ 0.75 ∼ 1.05(3H,m), 1.05 ∼ 2.87(26H,m), 3.37 ∼ 3.96(1H,m), 3.64(3H,s), 4.28 ∼ 4.53(0.5H,m), 4.77 ∼ 5.32(1.5H,m).

### Example 4

### Preparation of 16(RS)-fluoro-15-keto-9(O)-methano-Δ^{6(9α)}-PGI₁ methyl ester:

### 4-1 Synthesis of (1S,5S,6S,7R)-6-(t-butyldimethylsiloxymethyl)-3-[4-carbomethoxy-1(EZ)-butenyl]-7-tetrahydropyranyloxy-bicyclo[3.3.0]octo-2-ene (2):

According to the same manner as in the Example 1, 1-1 the title compound (2) was prepared.

### 4-2 Synthesis of (1S,5S,6S,7R)-6-(t-butyldimethylsiloxymethyl)-3-(4-carbomethoxybutyl)-7-tetrahydropyranyloxybicyclo[3.3.0]octo-2-ene (2'):

(1S,5S,6S,7R)-6-(t-Butyldimethylsiloxymethyl)-3-[4-carbomethoxy-1(EZ)-butenyl]-7-tetrahydropyranyloxy-bicyclo[3.3.0]octo-2-ene (2) (0.214 g) was dissolved in methanol, to which palladium (10%)/carbon (0.050 g) was added, and the mixture was stirred under hydrogen atmosphere at room temperature for 45 minutes. The reaction mixture was filtered and the filtrate was concentrated under reduced pressure. A crude product was chromatographed (hexane/ethyl acetate = 40/1 - 30/1) using silica gel treated with silver nitrate (10 wt. %) to give (1S,5S,6S,7R)-6-(t-butyldimethylsiloxymethyl)-3-(4-carbomethoxybutyl)-7-tetrahydropyranyloxybicyclo[3.3.0]octo-2-ene (2') as a colorless oily product. Yield: 0.151 g (70 %)
¹H NMR(CDCl₃)δ 0.05(6H,s), 0.88(9H,s), 0.97 ∼ 3.03(21H,m), 3.23 ∼ 4.15(5H,m), 3.62(3H,s), 4.45 ∼ 4.69(1H,m), 5.10 ∼ 5.33(1H,m).

### 4-3 Synthesis of (1S,5S,6S,7R)-3-(4-carbomethoxybutyl)-6-hydroxymethyl-7-tetrahydropyranyloxybicyclo[3.3.0]octo-2-ene (3'):

(1S,5S,6S,7R)-6-(t-Butyldimethylsiloxymethyl)-3-(4-carbomethoxybutyl)-7-tetrahydropyranyloxybicyclo[3.3.0]octo-2-ene (2') (0.294 g) was dissolved in THF, into which tetra-n-butyl-ammonium fluoride solution in THF (1.1 M, 2.2 ml) was added, and stirred at room temperature for 18 hours. A crude compound obtained after a usual work-up was purified on column chromatography (hexane/ethyl acetate = 1/1) to give (1S,5S,6S,7R)-3-(4-carbomethoxybutyl)-6-hydroxymethyl-7-tetrahydropyranyloxybicyclo[3.3.0]octo-2-ene (3') as a colorless oily product. Yield: 0.228 g
¹H NMR(CDCl₃)δ 0.76 ∼ 3.13(22H,m), 3.27 ∼ 4.13(5H,m), 3.63(3H,s), 4.46 ∼ 4.77(1H,m), 5.02 ∼ 5.42(1H,m).

### 4-4 Synthesis of (1S,5S,6S,7R)-3-(4-carbomethoxybutyl)-6-[4(RS)-fluoro-3-oxo-(E)-1-octenyl]-7-tetrahydoropyranyloxybicyclo[3.3.0]octo-2-ene (5'):

(1S,5S,6S,7R)-3-(4-Carbomethoxybutyl)-6-hydroxymethyl-7-tetrahydropyranyloxybicyclo[3.3.0]octo-2-ene (3') ((0.125 g) was dissolved in DMSO, to which a solution of triethylamine (0.93 ml) and sulfur trioxide/pyridine complex (0.504 g) in DMSO was added, and stirred at room temperature for 1.5 hours. A crude aldehyde (4') obtained after usual work-up was dissolved in THF, and reacted at 50 °C with the anion prepared from dimethyl(2-oxo-3-fluoroheptyl)phosphonate (0.341 g) and sodium hydride. After stirred for 3 hours, the reaction mixture was neutralized with acetic acid. A crude product obtained according to a usual work-up was purified on column chromatography (hexane/ethyl acetate = 7/1) to give (1S,5S,6S,7R)-3-(4-carbomethoxybutyl)-6-[4(RS)-fluoro-3-oxo-(E)-1-octenyl]-7-tetrahydoropyranyloxybicyclo[3.3.0]octo-2-ene (5'). Yield: 0.088 g (56%)
¹H NMR(CDCl₃)δ 0.75 ∼ 1.06(3H,m), 1.05 ∼ 3.14(27H,m), 3.26 ∼ 4.13(3H,m), 3.63(3H,s), 4.38 ∼ 4.71(1.5H,m), 5.01 ∼ 5.43(1.5H,m) 6.26 ∼ 6.68(1H,m), 6.80 ∼ 7.26(1H,m).

### 4-5 Synthesis of 16(RS)-fluoro-15-keto-9(O)-methano-Δ^{6(9α)}-PGI₁ methyl ester (7'):

(1S,5S,6S,7R)-3-(4-Carbomethoxybutyl)-6-[4(RS)-fluoro-3-oxo-(E)-1-octenyl]-7-tetrahydoropyranyloxybicyclo [3.3.0]octo-2-ene (5') (0.088 g) was dissolved in a mixture of acetic acid, water and THF (4 : 2 : 1), and stirred at 45 °C for 3 hours. The reaction mixture was concentrated under reduced pressure, and a crude product obtained was purified on column chromatography (hexane/ethyl acetate = 3/1) to give 16(RS)-fluoro-15-keto-9(O)-methano-Δ^{6(9α)}-PGI₁ methyl ester (7'). Yield: 0.069 g (96%)
¹H NMR(CDCl₃)δ 0.72 ∼ 1.04(3H,m), 1.04 ∼ 3.18(22H,m), 3.62(3H,s), 3.70 ∼ 4.12(1H,m) 4.43 ∼ 4.63(0.5H,m), 4.98 ∼ 5.23(0.5H,m), 5.18 ∼ 5.35(1H,m), 6.53(1H,dd,J = 16 Hz,J = 3 Hz), 6.98(1H,dd,J = 16 Hz,J = 9 Hz).

### Example 5

### Preparation of 16,16-difluoro-15-keto-9(O)-methano-Δ^{6(9α)}-PGI₁ methyl ester:

### 5-1 Synthesis of (1S,5S,6S,7R)-3-(4-carbomethoxybutyl)-6-[4,4-difluoro-3-oxo-(E)-1-octenyl]-7-tetrahydropyranyloxybicyclo[3.3.0]octo-2-ene (8):

(1S,5S,6S,7R)-3-(4-Carbomethoxybutyl)-6-hydroxymethyl-7-tetrahydropyranyloxybicyclo[3.3.0]octo-2-ene (3') (0.108 g) was dissolved in DMSO, to which a solution of triethylamine (0.90 ml) and sulfur trioxide/pyridine complex (0.488 g) in DMSO was added, and stirred at room temperature for 30 minutes. A usual work-up gave a crude aldehyde. The crude aldehyde was dissolved in THF, and reacted with an anion prepared from dimethyl(2-oxo-3,3-difluoroheptyl)phosphate (0.435 g) and sodium hydride. The mixture was heated for 48 hours under reflux, and then neutralized with acetic acid. A crude product obtained after a usual work-up was purified on column chromatography (hexane/ethyl acetate = 7/1) to give (1S,5S,6S,7R)-3-(4-carbomethoxybutyl)-6-[4,4-difluoro-3-oxo-(E)-1-octenyl]-7-tetrahydropyranyloxybicyclo[3.3.0]octo-2-ene (8) as a colorless oily product. Yield: 0.091 g (64%)
¹H NMR(CDCl₃)δ 0.76 ∼ 1.05(3H,m), 1.05 ∼ 3.17(27H,m), 3.25 ∼ 4.15(3H,m), 3.63(3H,s), 4.35 ∼ 4.75(1H,m), 5.09 ∼ 5.37(1H,m), 6.56(1H,dd,J = 15 Hz,J = 6 Hz), 6.86 ∼ 7.37(1H,m).

### 5-2 Synthesis of 16,16-difluoro-15-keto-9(O)-methano-Δ^{6(9α)}-PGI₁ methyl ester (9):

(1S,5S,6S,7R)-3-(4-Carbomethoxybutyl)-6-[4,4-difluoro-3-oxo-(E)-1-octenyl]-7-tetrahydropyranyloxy-bicyclo[3.3.0]octo-2-ene (8) (0.091 g) was dissolved in a mixture of acetic acid, water and THF (4 : 2 : 1), and stirred at 45 °C for 3 hours. The reaction mixture was concentrated under reduced pressure, and a crude product was purified on column chromatography (hexane/ethyl acetate = 2/1) to give 16,16-difluoro-15-keto-9(O)-methano-Δ^{6(9α)}-PGI₁ methyl ester (9) as a colorless oily product. Yield: 0.060 g (80%)
¹H NMR(CDCl₃)δ 0.76 ∼ 1.05(3H,m), 1.05 ∼ 3.21(22H,m), 3.62(3H,s), 3.73 ∼ 4.17(1H,m), 5.09 ∼ 5.43(1H,m), 6.56(1H,d,J = 15 Hz), 7.12(1H,dd,J = 15 Hz,J = 7.5 Hz).

### Example 6

### Preparation of 16(RS)-fluoro-15-keto-9(O)-methano-PGI₂:

### 6-1 Synthesis of (1S,5S,6S,7R)-3-[4-carbomethoxy-1(EZ)-butenyl]-6-hydroxymethyl-7-tetrahydropyranyloxy-bicyclo[3.3.0]octo-2-ene (3):

According to the same manner as in 1-2 of the Example 1 the Compound (3) was prepared.

### 6-2 Synthesis of (1S,5S,6S,7R)-3-[4-carbomethoxy-1(EZ)-butenyl]-6-[4(RS)-fluoro-3-oxo-(E)-1-octenyl]-7-tetrahydropyranyloxy-bicyclo[3.3.0]octo-2-ene (5):

According to the same manner as in 1-3 of the Example 1 the above Compound (5) was prepared.

### 6-3 synthesis of (1S,5S,6S,7R)-3-[4-carbomethoxy-1(EZ)-butenyl]-6-[4(RS)-fluoro-3(RS)-hydroxy-(E)-1-octenyl]-7-tetrahydropyranyloxybicyclo[3.3.0]octo-2-ene (5''):

(1S,5S,6S,7R)-3-[4-Carbomethoxy-1(EZ)-butenyl]-6-[4(RS)-fluoro-3-oxo(E)-1-octenyl]-7-tetrahydropyranyloxy-bicyclo[3.3.0]octo-2-ene (5) (0.088g) was dissolved in methanol, into which sodium borohydride (0.008 g) was added at 0 °C and stirred for 30 minutes. The reaction mixture was treated by a usual manner to give (1S,5S,6S,7R)-3-[4-carbomethoxy-1(EZ)-butenyl]-6-[4(RS)-fluoro-3(RS)-hydroxy-(E)-1-octenyl]-7-tetrahydropyranyloxybicyclo[3.3.0]octo-2-ene (5'') as a colorless oily product. Yield: 0.089 g
¹H NMR(CDCl₃)δ 0.67 ∼ 1.03(3H,m), 1.03 ∼ 3.19(24H,m), 3.22 ∼ 4.34(4.5H,m), 3.62(3H,s), 4.40 ∼ 4.74(1.5H,m), 5.07 ∼ 6.32(5H,m).

### 6-4 Synthesis of (1S,2S,3R,5S)-(E)-7-(4-carbomethoxybutylidene)-2-[4(RS)-fluoro-3(RS)-hydroxy(E)-1-octenyl]-3-tetrahydropyranyloxybicyclo[3.3.0]octane (12'):

(1S,5S,6S,7R)-3-[4-Carbomethoxy-1(EZ)-butenyl]-6-[4(RS)-fluoro-3(RS)-hydroxy-(E)-1-octenyl]-7-tetrahydropyranyloxybicyclo[3.3.0]octo-2-ene (5'') (0.089 g) was put in an autoclave and dissolved in acetone, into which tricarbonyl chromium/methyl benzoate complex (0.011 g) was added, and then degassed. The mixture in the autoclave was stirred under hydrogen pressure of 70 kg/cm² at 120 °C for 15 hours. The reaction mixture was concentrated under reduced pressure, and the obtained crude product was purified on column chromatography (hexane/ethyl acetate = 2/1) to give (1S,2S,3R,5S)-(E)-7-(4-carbomethoxybutylidene)-2-[4(RS)-fluoro-3(RS)-hydroxy(E)-1-octenyl]-3-tetrahydropyranyloxybicyclo[3.3.0]octane (12') as a colorless oily product. Yield: 0.078 g (87%)
¹H NMR(CDCl₃)δ 0.70 ∼ 1.04(3H,m), 1.04 ∼ 2.67(28H,m), 3.21 ∼ 4.32(4.5H,m), 4.36 ∼ 4.75(1.5H,m), 4.99 ∼ 5.30(1H,m), 5.30 ∼ 5.92(20,m).

### 6-5 Synthesis of (1S,2S,3R,5S)-(E)-7-(4-Carboxybutylidene)-2-[4(RS)-fluoro-3(RS)-hydroxy-(E)-1-octenyl]-3-tetrahydropyranyloxybicyclo[3.3.0]octane (13'):

(1S,2S,3R,5S)-(E)-7-(4-Carbomethoxybutylidene)-2-[4(RS)-fluoro-3(RS)-hydroxy(E)-1-octenyl]-3-tetrahydropyranyloxybicyclo[3.3.0]octane (12') (0.129 g) was dissolved in methanol, into which 1N aqueous solution of sodium hydroxide (2 ml) was added, and stirred at room temperature for 6 hours. After a usual work-up (1S,2S,3R,5S)-(E)-7-(4-carboxybutylidene)-2-[4(RS)-fluoro-3(RS)-hydroxy-(E)-1-octenyl]-3-tetrahydropyranyloxybicyclo [3.3.0]octane (13') was obtained as a colorless oily product. Yield: 0.140 g
¹H NMR(CDCl₃)δ 0.70 ∼ 1.05(3H,m), 1.05 ∼ 2.70(27H,m), 3.26 ∼ 6.06(10H,m).

### 6-6 Synthesis of (1S,2S,3R,5S)-(E)-7-(4-carboxybutylidene)-2-[4(RS)-fluoro-3-oxo-(E)-1-octenyl]-3-tetrahydropyranyloxybicyclo[3.3.0]octane (14'):

(1S,2S,3R,5S)-(E)-7-(4-Carboxybutylidene)-2-[4(RS)-fluoro-3(RS)-hydroxy-(E)-1-octenyl]-3-tetrahydropyranyloxybicyclo[3.3.0)octane (13') (0.140 g) was subjected to Jones oxidation between -15 °C and -20 °C. The mixture was stirred for 30 minutes, isopropyl alcohol was added, and the resultant was treated by a usual work-up. The obtained crude product was purified on column chromatography (hexane/ethyl acetate = 6/1 - 5/1) to give (1S,2S,3R,5S)-(E)-7-(4-carboxybutylidene)-2-[4(RS)-fluoro-3-oxo-(E)-1-octenyl]-3-tetrahydropyranyloxybicyclo[3.3.0]octane (14') as a colorless oily product. Yield: 0.106 g (76%)
¹H NMR(CDCl₃)δ 0.75 ∼ 1.04(3H,m), 1.04 ∼ 2.78(27H,m), 3.23 ∼ 4.14(3H,m), 4.37 ∼ 4.73(1.5H,m), 5.02 ∼ 5.36(1.5H,m), 6.32 ∼ 6.67(1H,m), 6.73 ∼ 7.26(1H,m).

### 6-7 Synthesis of 16(RS)-fluoro-15-keto-9(O)-methano-PGI₂ (15'):

(1S,2S,3R,5S)-(E)-7-(4-Carboxybutylidene)-2-[4(RS)-fluoro-3-oxo-(E)-1-octenyl]-3-tetrahydropyranyloxybicyclo[3.3.0]octane (14') (0.106 g) was dissolved in a mixture of acetic acid, water and THF (4 : 2 : 1), and stirred at 45 °C for 3.5 hours. The reaction mixture was concentrated under reduced pressure. The crude product was purified on column chromatography (hexane/ethyl acetate = 6/1 - 2/1) using silica gel (CC-4: available from Mallineckrodt Co., Ltd.) to give 16(RS)-fluoro-15-keto-9(O)-methano-PGI₂ (15') as a colorless oily product. Yield: 0.047 g (52%)
¹H NMR(CDCl₃)δ 0.74 ∼ 1.04(3H,m), 1.04 ∼ 2.80(21H,m), 3.67 ∼ 4.07(1H,m), 4.43 ∼ 4.65(0.5H,m), 4.99 ∼ 5.37(1.5H,m), 4.00 ∼ 5.60(2H,brs), 6.51(1H,dd,J = 17 Hz, J = 4 Hz), 6.94(1H,dd,J = 17 Hz,J = 7 Hz).

### Example 7

### Preparation of 16,16-difluoro-15-keto-9(O)-methano-PGI₂:

### 7-1 Synthesis of (1S,5S,6S,7R)-3-[4-carbomethoxy-1(EZ)-butenyl]-6-[4,4-difluoro-3-oxo-(E)-1-octenyl]-7-tetrahydropyranyloxybicyclo[3.3.0]octo-2-ene (16):

(1S,5S,6R,7R)-3-[4-carbomethoxy-1(EZ)-butenyl]-6-hydroxymethyl-7-tetrahydropyranyloxy-bicyclo[3.3.0]octo-2-ene(3) (0.333 g) was subjected to Collins oxidation in methylene chloride at 0 °C. After 30 minutes sodium hydrogen sulfate was added into the reaction mixture, and filtrated. The filtrate was concentrated under reduced pressure to give crude aldehyde (4), which was dissolved in THF, and reacted at 70 °C with an anion prepared from dimethyl(2-oxo-3,3-difluoroheptyl)phosphonate (0.970 g) and sodium hydride. After stirred for 17 hours, the reaction product was neutralized by acetic acid. A crude product obtained after a usual work-up was purified by column chromatography (hexane/ethyl acetate = 6/1) to give (1S,5S,6S,7R)-3-[4-carbomethoxy-1(EZ)-butenyl]-6-[4,4-difluoro-3-oxo-(E)-1-octenyl]-7-tetrahydropyranyloxy-bicyclo[3.3.0]octo-2-ene (16) as a colorless oily product. Yield: 0.196 g (43%)
¹H NMR(CDCl₃)δ 0.73 ∼ 1.06(3H,m), 1.04 ∼ 2.90(23H,m), 2.90 ∼ 4.17(3H,m), 3.63(3H,s), 4.33 ∼ 4.71(1H,m), 5.10 ∼ 5.66(2H,m), 5.94(0.67H,d,J = 12 Hz), 6.22(0.33H,d,J = 16.5 Hz), 6.57(1H,dd,J = 15 Hz,J = 6 Hz), 6.86 ∼ 7.33(1H,m).

### 7-2 Synthesis of (1S,5S,6S,7R)-3-[4-carbomethoxy-1(EZ)-butenyl]-6-[4,4-difluoro-3(RS)hydroxy-(E)-1-octenyl]- 7-tetrahydropyranyloxy-bicyclo[3.3.0]octo-2-ene (17):

(1S,5S,6S,7R)-3-[4-Carbomethoxy-1(EZ)-butenyl]-6-[4,4-difluoro-3-oxo-(E)-1-octenyl]-7-tetrahydropyranyloxy-bicyclo[3.3.0]octo-2-ene (16) (0.196 g) was dissolved in methanol, to which sodium borohydride (0.015 g) was added at 0 °C, and stirred for 30 minutes. After a usual work-up (1S,5S,6S,7R)-3-[ 4-carbomethoxy-1(EZ)-butenyl]-6-[4,4-difluoro-3(RS)hydroxy-(E)-1-octenyl]-7-tetrahydropyranyloxy-bicyclo[3.3.0]octo-2-ene (17) was obtained as a colorless oily product. Yield: 0.184 g (93%)
¹H NMR(CDCl₃)δ 0.70 ∼ 1.03(3H,m), 1.03 ∼ 2.72(24H,m), 2.85 ∼ 3.23(1H,m), 3.23 ∼ 3.96(2H,m), 3.63(3H,s), 3.96 ∼ 4.35(1H,m), 4.46 ∼ 4.68(1H,m), 5.05 ∼ 6.35(5H,m).

### 7-3 Synthesis of (1S,2S,3R,5S)-(E)-7-(4-carbomethoxybutylidene)-2-[4,4-difluoro-3(RS)-hydroxy-(E)-1-octenyl]-3-tetrahydropyranyloxybicyclo[3.3.0]octane (18):

(1S,5S,6S,7R)-3-[4-Carbomethoxy-1(EZ)-butenyl]-6-[4,4-difluoro-3(RS)hydroxy-(E)-1-octenyl]-7-tetrahydropyranyloxybicyclo[3.3.0]octo-2-ene (17) (0.184 g) was dissolved in acetone and put in an autoclave, into which tricarbonyl chromium/methyl benzoate complex (0.021 g) was added, and then degassed. The mixture in the autoclave was stirred under hydrogen pressure (70 kg/cm²) at 120 °C for 15 hours. The reaction mixture was concentrated under reduced pressure, and the obtained crude product was purified on column chromatography (hexane/ethyl acetate = 7/2 - 3/1) to give (1S,2S,3R,5S)-(E)-7-(4-carbomethoxybutylidene)-2[4,4-difluoro-3(RS)-hydroxy-(E)-1-octenyl]-3-tetrahydropyranyloxybicyclo[3.3.0]octane (18) as a colorless oily product. Yield: 0.175 g (95%)
¹H NMR(CDCl₃)δ 0.75 ∼ 1.05(3H,m), 1.05 ∼ 2.63(28H,m), 3.23 ∼ 4.00(3H,m), 3.62(3H,s), 4.00 ∼ 4.40(1H,m), 4.48 ∼ 4.66(1H,m), 5.03 ∼ 5.32(1H,m), 5.33 ∼ 6.05(2H,m).

### 7-4 Synthesis of (1S,2S,3R,5S)-(E)-7-(4-carboxybutylidene)-2-[4,4-difluoro-3(RS)-hydroxy-(E)-1-octenyl]-3-tetrahydropyranyloxybicyclo[3.3.0]octane (19):

(1S,2S,3R,5S)-(E)-7-(4-Carbomethoxybutylidene)-2-[4,4-difluoro-3(RS)-hydroxy-(E)-1-octenyl]-3-tetrahydropyranyloxybicyclo[3.3.0]octane (18) (0.175 g) was dissolved into methanol, to which 1N aqueous solution of sodium hydroxide was added, and the mixture was stirred until the mixture became completely clear. After a usual work-up a crude product, (1S,2S,3R,5S)-(E)-7-(4-carboxybutylidene)-2-[4,4-difluoro-3(RS)-hydroxy-(E)-1-octenyl]-3-tetrahydropyranyloxybicyclo[3.3.0]octane (19), was obtained. Yield: 0.172 g
¹H NMR (CDCl₃)δ 0.70 ∼ 1.03(3H,m), 1.03 ∼ 2.73(27H,m), 3.22 ∼ 4.39(4H,m), 4.40 ∼ 4.72(1H,m), 4.98 ∼ 5.35(1H,m), 5.35 ∼ 6.03(2H,m), 3.22 ∼ 6.13(2H,brs).

### 7-5 Synthesis of (1S,2S,3R,5S)-(E)-7-(4-carboxybutylidene)-2-[4,4-difluoro-3-oxo-(E)-1-octenyl]-3- tetrahydropyranyloxybicyclo[3.3.0]octane (20):

(1S,2S,3R,5S)-(E)-7-(4-Carboxybutylidene)-2-[4,4-difluoro-3(RS)-hydroxy-(E)-1-octenyl]-3-tetrahydropyranyloxybicyclo[3.3.0]octane (19) (0.172 g) was subjected to Collins oxidation at room temperature. The mixture was stirred for 30 minutes, sodium hydrogen sulfonate was added, and then filtered. The filtrate was concentrated. The obtained crude product was purified on column chromatography (hexane/ethyl acetate = 20/1 - 10/1) using a silica gel (CC-4) to give (1S,2S,3R,5S)-(E)-7-(4-carboxybutylidene)-2-[4,4-difluoro-3-oxo-(E)-1-octenyl]-3-tetrahydropyranyloxy-bicyclo[3.3.0]octane (20). Yield: 0.050 g (30%)
¹H NMR(CDCl₃)δ 0.66 ∼ 1.03(3H,m), 1.03 ∼ 2.75(27H,m), 3.24 ∼ 4.08(3H,m), 4.36 ∼ 4.68(1H,m), 5.07 ∼ 5.36(1H,m), 6.52(1H,dd,J = 15 Hz, J = 6 Hz), 6.83 ∼ 7.30(1H,m), 7.20 ∼ 8.20(1H,brs).

### 7-6 Synthesis of 16,16-difluoro-15-keto-9(O)-methano-PGI₂ (21):

(1S,2S,3R,5S)-(E)-7-(4-Carboxybutylidene)-2-[4,4-difluoro-3-oxo-(E)-1-octenyl]-3-tetrahydropyranyloxybicyclo[3.3.0]octane (20) (0.050 g) was dissolved in a mixture of acetic acid, water and THF (4 : 2 : 1), and stirred at 45 °C for 3.5 hours. The reaction mixture was concentrated under reduced pressure, and the obtained crude product was purified on column chromatography (hexane/ethyl acetate = 4/1) using a silica gel (CC-4) to give 16,16-difluoro-15-keto-9(O)-methano-PGI₂ (21) as a colorless oily product. Yield: 0.033 g (80%)
¹H NMR(CDCl₃)δ 0.70 ∼ 1.05(3H,m), 1.05 ∼ 2.90(21H,m), 3.65 ∼ 4.20(1H,m), 5.05 ∼ 5.40(1H,m), 4.80 ∼ 5.95(2H,brs), 6.53(1H,d,J = 16 Hz), 7.07(1H,dd,J = 16 Hz, J = 7.5 Hz).

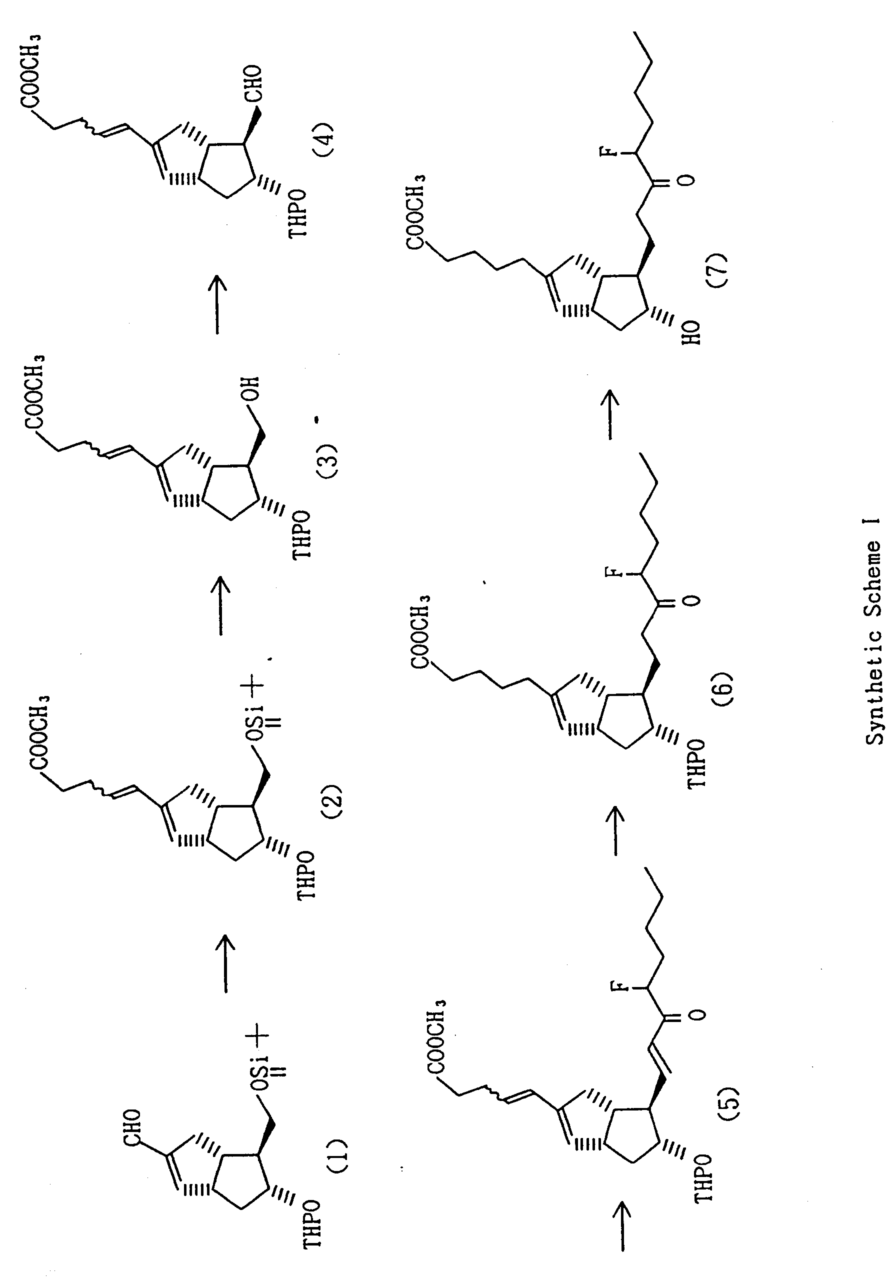

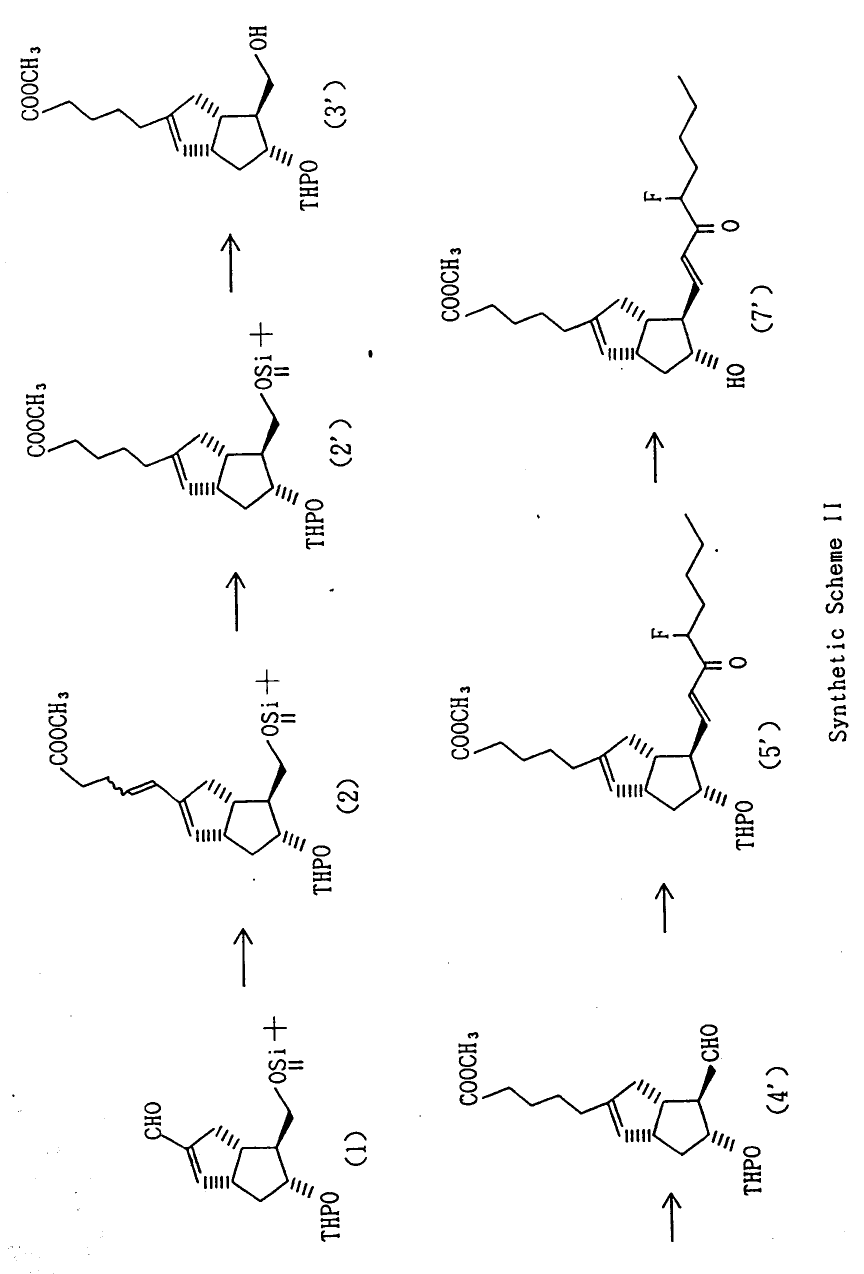

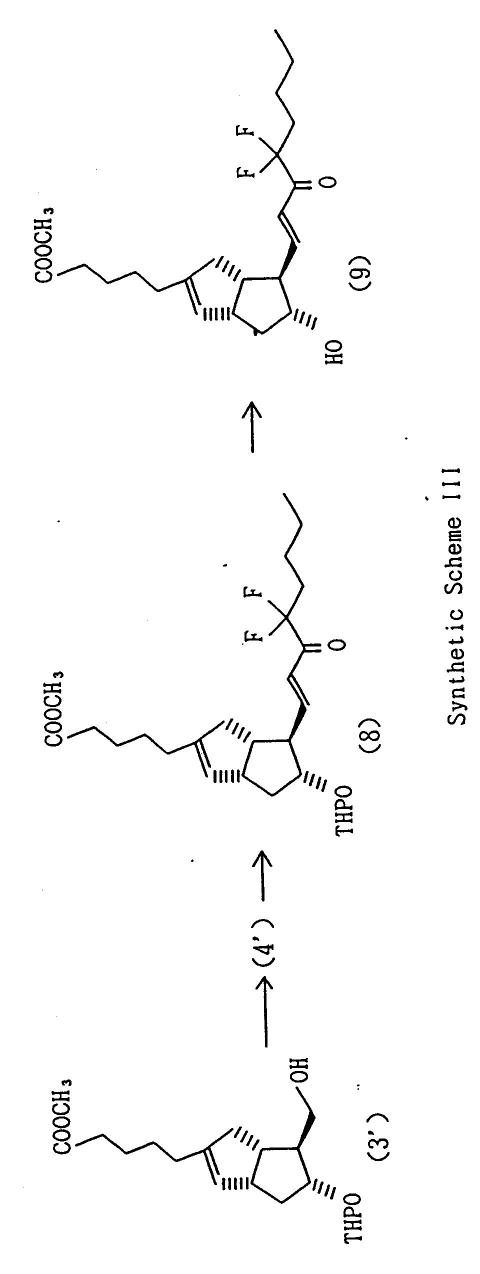

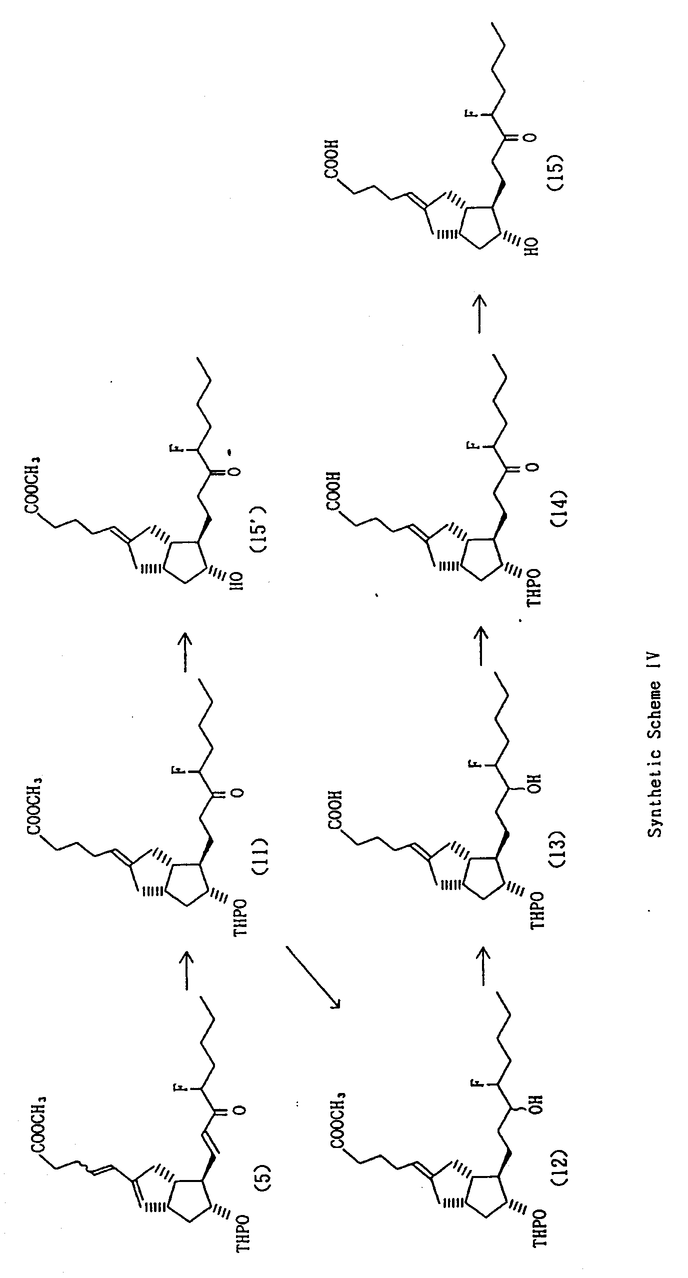

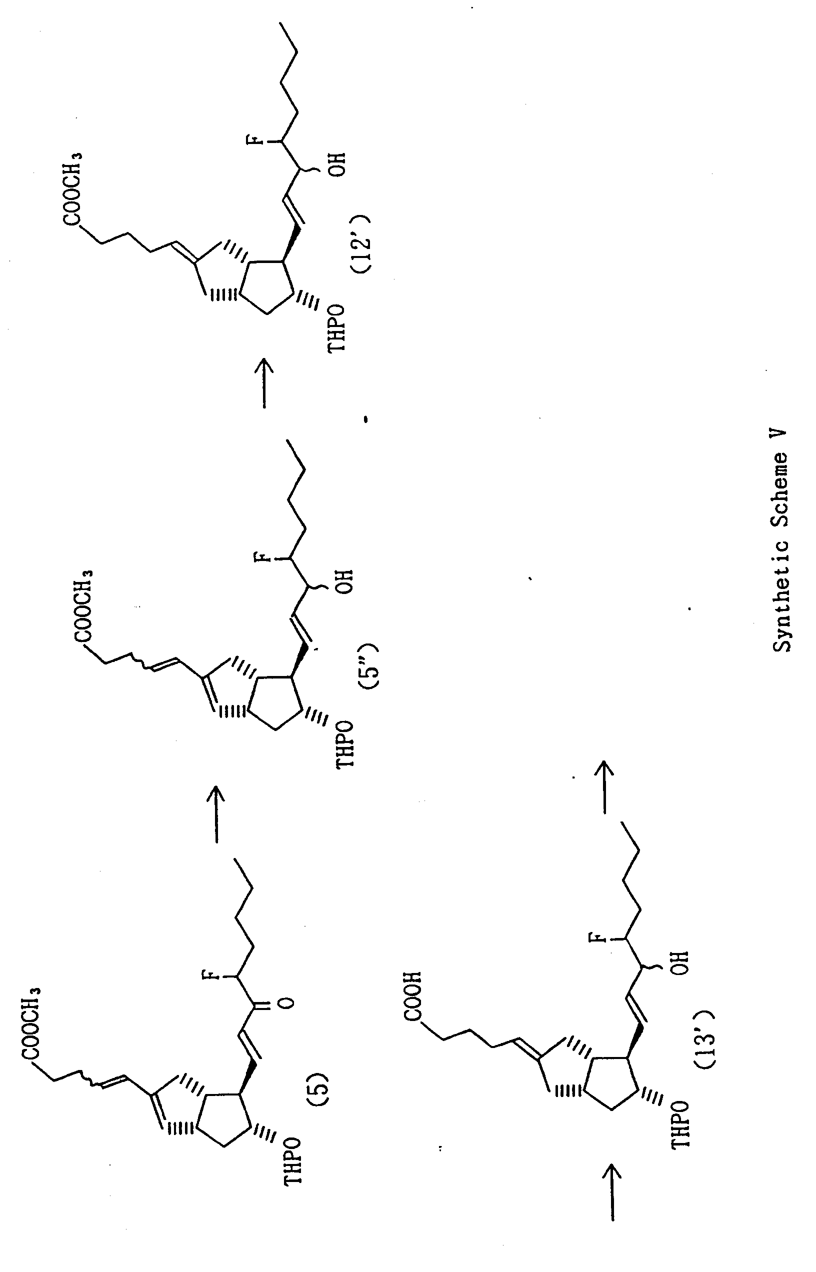

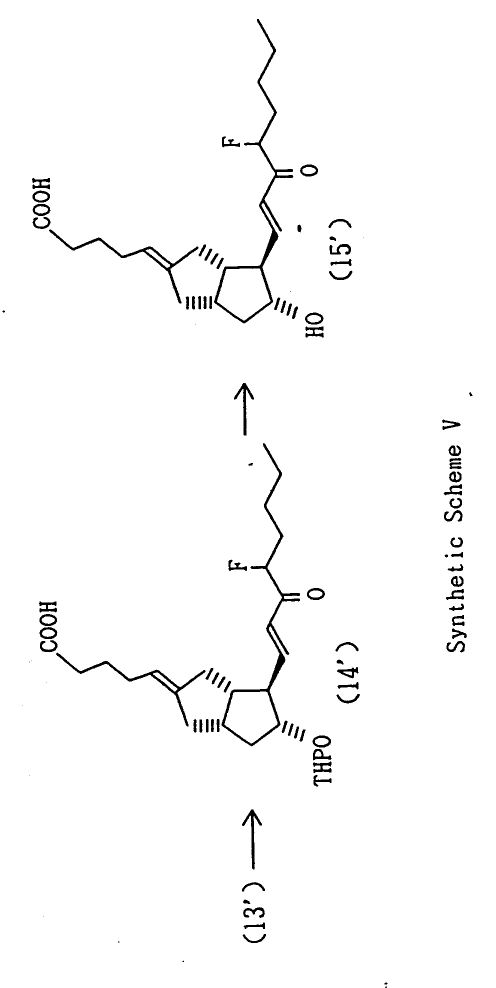

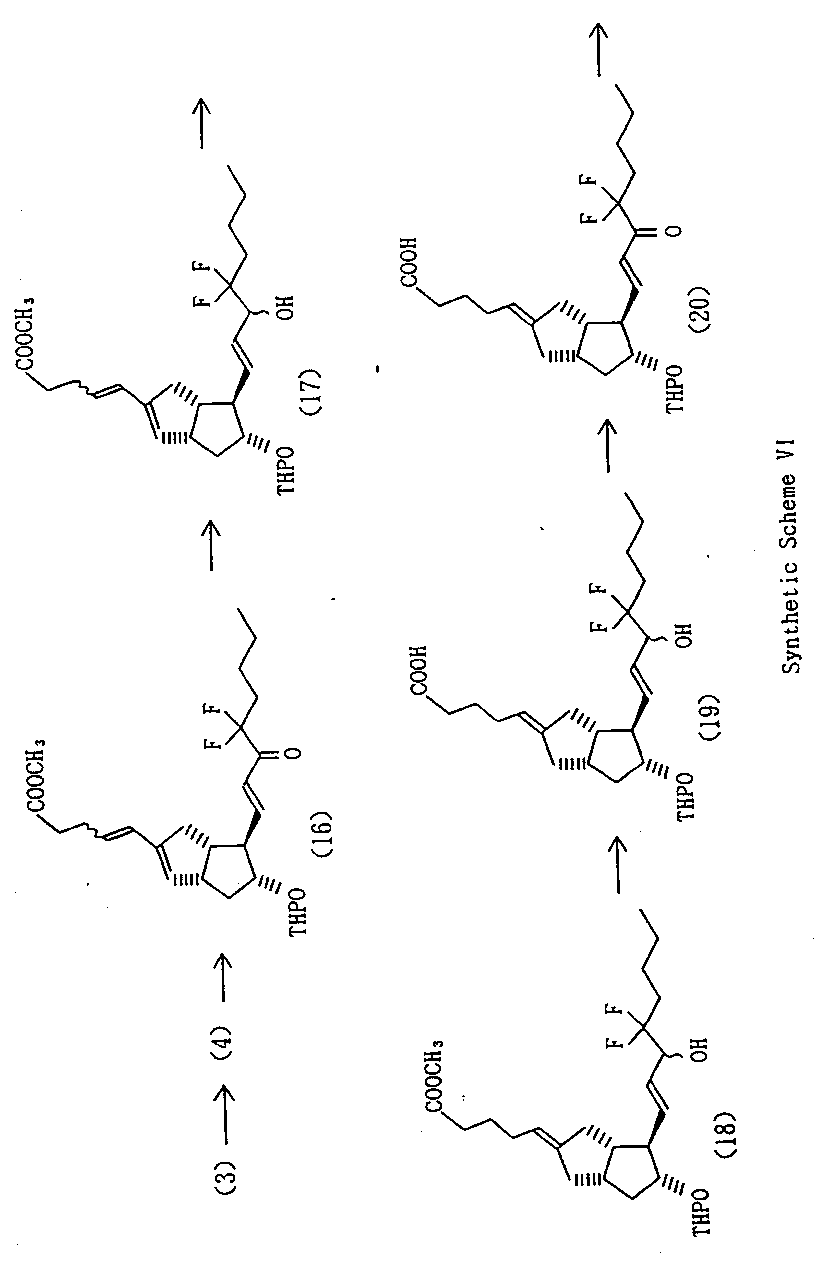

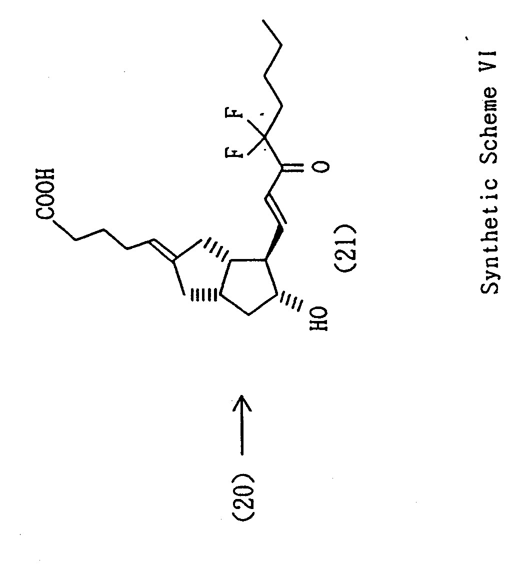

## Claims

1. Prostaglandin Is represented by following formula (I) and (II): 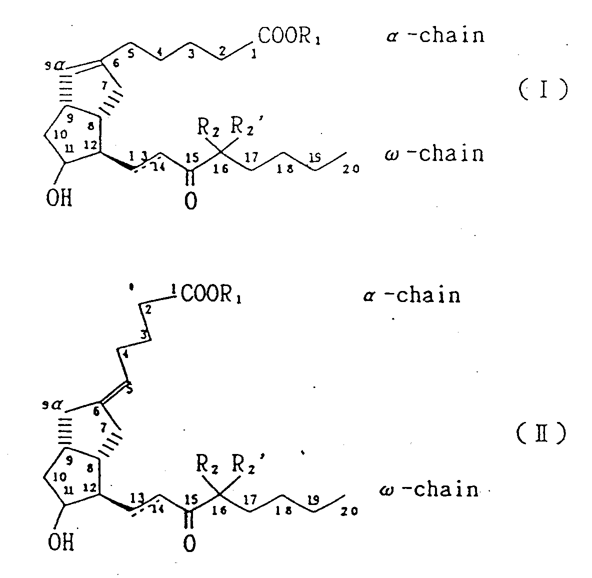
wherein R1 is a hydrogen ator or an alkyl group;
R2 is a fluorine atom;
R2' is a hydrogen atom, a fluorine atom, an alkyl group, a phenyl group, a benzyl group, a hydroxyl group or an alkoxy group; or salt thereof.

2. Prostaglandin Is of Claim 1 in which R2' is a fluorine atom.

3. Prostaglandin Is of Claim 1 being selected from 13,14-dihydro-15-keto-16(RS)- fluoro-9(O)-methano-Δ^{6(9α)}-PGI₁, 16(RS)-fluoro-15-keto-9(O)-methano-Δ^{6(9α)}-PGI₁, 16,16-difluoro-15-keto-9(O)-methano-Δ^{6(9α)}-PGI₁, an alkyl ester, or a salt thereof.

4. Prostaglandin Is of Claim 1 being selected from 13,14-dihydro-15-keto-16,16-difluoro-9(O)-methano-PGI₂, 16(RS)-fluoro-15-keto-9(O)-methano-PGI₂, 16,16-difluro-15-keto-9(O)-methano-PGI₂, an alkyl ester or a salt thereof.

## Patentansprüche

1. Prostaglandin Is, dargestellt durch die folgenden Formeln (I) und (II): 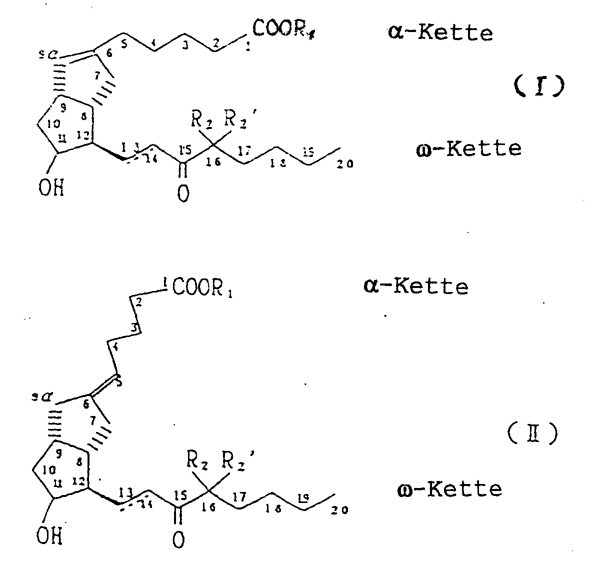 worin
R₁ ein Wasserstoffatom oder eine Alkylgruppe bedeutet;
R₂ ein Fluoratom bedeutet,
R₂' ein Wasserstoffatom, ein Fluoratom, eine Alkylgruppe, eine Phenylgruppe, eine Benzylgruppe, eine Hydroxylgruppe oder eine Alkoxygruppe bedeutet,
oder ein Salz davon.

2. Prostaglandin Is nach Anspruch 1, dadurch **gekennzeichnet,** daß R₂' ein Fluoratom bedeutet.

3. Prostaglandin Is nach Anspruch 1, ausgewählt aus 13,14-Dihydro-15-keto-16(RS)-fluor-9(O)-methano-Δ^{6(9α)}-PGI₁, 16(RS)-Fluor-15-keto-9(O)-methano-Δ^{6(9α)}-PGI₁, 16,16-Difluor-15-keto-9(O)-methano-Δ^{6(9α)}-PGI₁, einem Alkylester oder Salz davon.

4. Prostaglandin Is nach Anspruch 1, ausgewählt aus 13,14-Dihydro-15-keto-16,16-difluor-9(O)-methano-PGI₂, 16(RS)-Fluor-15-keto-9(O)-methano-PGI₂, 16,16-Difluor-15-keto-9(O)-methano-PGI₂, einem Alkylester oder einem Salz davon.

## Revendications

1. Prostaglandine Is représentée par les formules (I) et (II) suivantes: 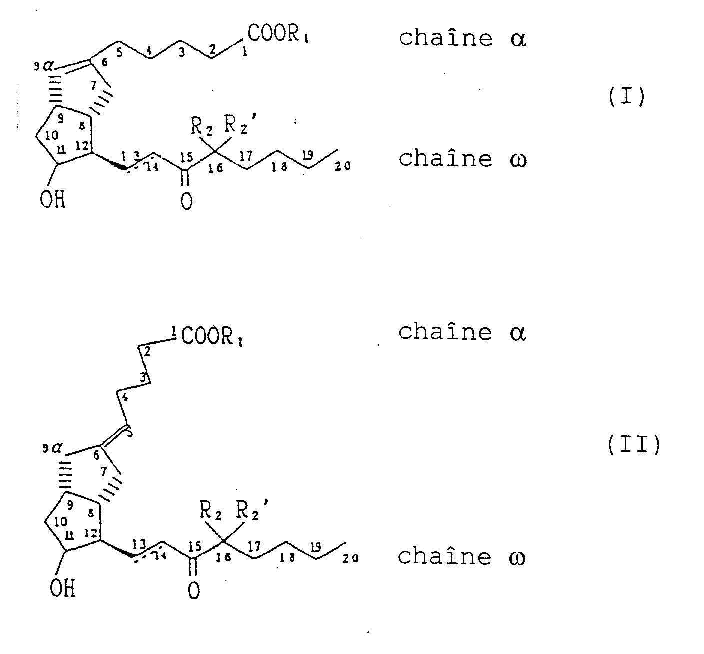 dans lesquelles
R₁ est un atome d'hydrogène ou un groupe alkyle;
R₂ est un atome de fluor;
R₂' est un atome d'hydrogène, un atome de fluor, un groupe alkyle, un groupe phényle, un groupe benzyle, un groupe hydroxyle ou un groupe alcoxy ;
ou un sel de celle-ci.

2. Prostaglandine Is selon la revendication 1 dans laquelle R₂' est un atome de fluor.

3. Prostaglandine Is selon la revendication 1, caractérisée en ce qu'elle est choisie parmi la 13, 14-dihydro-15-céto-16(RS)-fluoro-9(0)-méthano-Δ^{6(9α)}-PGI₁, la 16(RS)-fluoro-15-céto-9(O)-méthano-Δ^{6(9α)}-PGI₁, la 16,16-difluoro-15-céto-9(O)-méthano-Δ^{6(9α)}-PGI₁, un ester alkylique, ou un sel de celles-ci.

4. Prostaglandine Is selon la revendication 1, caractérisée en ce qu'elle est choisie parmi la 13,14-dihydro-15-céto-16,16-difluoro-9(O)-méthano-PGI_{2,} la 16(RS)-fluoro-15-céto-9(O)-méthano-PGI₂, la 16,16-difluoro-15-céto-9(O)-méthano-PGI₂, un ester alkylique ou un sel de celles-ci.
